# EUROPEAN PATENT APPLICATION

(11) **EP 4 721 748 A1**
(43) Date of publication of application: **08.04.2026**
(21) Application number: 25163861.5
(22) Date of filing: 14.03.2025
(51) Int. Cl.: A61K 31/7125, A61K 31/4745, A61K 9/00, A61K 45/06, A61P 35/00

(54) **SEX-SPECIFIC TOLL-LIKE RECEPTOR (TLR) AGONIST THERAPY FOR THE TREATMENT OF EARLY-STAGE MELANOMA**

(71) Applicant: Stichting Amsterdam UMC, 1081 HV Amsterdam (NL)
(72) Inventor: de Gruijl, Tanja, 1105 AZ Amsterdam (NL); Koster, Bas, 1105 AZ Amsterdam (NL)
(74) Representative: de Pauw, Elmar Sebastian David

(57) **Abstract**

The invention relates to a Toll-like receptor (TLR) agonist selected from a TLR9 agonist for use in the treatment of early-stage melanoma in a male patient, wherein the treatment comprises intradermal administration of the TLR9 agonist at the primary tumor excision site prior to sentinel lymph node biopsy (SNB).

The invention further relates to a Toll-like receptor (TLR) agonist selected from a TLR7/8 agonist, alone or in combination with a TLR9 agonist, for use in the treatment of early-stage melanoma in a female patient, wherein the treatment comprises intradermal administration of the agonist(s) at the primary tumor excision site prior to sentinel lymph node biopsy (SNB).

## Description

### FIELD OF THE INVENTION

The present invention relates to the use of Toll-like receptor (TLR) agonists in the treatment of early-stage melanoma, particularly for enhancing immune activation prior to sentinel lymph node biopsy (SNB). More specifically, the invention provides a sex-specific immunotherapy approach using TLR9 agonists, TLR7/8 agonists, or a combination thereof, based on patient-specific immune response characteristics.

### BACKGROUND

Melanoma is an aggressive form of skin cancer with a high potential for metastasis. Early-stage melanoma is typically treated through surgical excision of the primary tumor, often followed by a sentinel lymph node biopsy (SNB) to assess the risk of metastatic spread. While surgery remains the standard of care, a significant number of patients experience disease recurrence despite complete tumor excision. This suggests a need for additional therapeutic approaches to enhance immune activation and reduce the risk of progression.

Toll-like receptor (TLR) agonists have been explored as immunotherapeutic agents due to their ability to stimulate the innate immune system and enhance antigen presentation. Among them, TLR9 agonists, such as CpG oligodeoxynucleotides, have been shown to activate dendritic cells, promote cytokine release, and enhance the priming of anti-tumor T cells. Similarly, TLR7/8 agonists, such as imidazoquinolines, have been demonstrated to induce strong inflammatory responses through activation of myeloid and plasmacytoid dendritic cells. Preclinical and early clinical studies suggest that the administration of TLR agonists in the context of melanoma treatment can enhance local and systemic anti-tumor immunity.

Several studies have investigated the use of TLR agonists in patients with early-stage melanoma, particularly in the context of administration prior to SNB. It has been reported that TLR9 agonists can activate immune cells within the tumor-draining lymph nodes, potentially improving the detection and clearance of residual tumor cells. However, while these studies suggest a beneficial role for TLR agonists in melanoma, variability in patient responses has been observed, indicating that not all individuals respond equally to the same treatment.

Although previous research has provided insights into the role of TLR agonists in early-stage melanoma, the variability in treatment responses highlights the need for a more refined understanding of immune modulation in this context. Further investigations are required to determine how different patient populations respond to TLR stimulation and whether specific factors influence the efficacy of these immunotherapies.

### SUMMARY OF THE INVENTION

The present invention relates to a Toll-like receptor (TLR) agonist for use in the treatment of early-stage melanoma, wherein the TLR agonist is selected based on the patient's sex and is administered intradermally at the primary tumor excision site prior to sentinel lymph node biopsy (SNB).

According to the invention, the TLR agonist is selected from a TLR9 agonist for use in a male patient or from a TLR7/8 agonist, alone or in combination with a TLR9 agonist, for use in a female patient.

Preferably, the TLR9 agonist is selected from agatolimod (CPG7909) or tilsotolimod (IMO-2125).

Preferably, when a TLR7/8 agonist is administered, the TLR7/8 agonist is resiquimod (R848).

In a preferred embodiment, a female patient receives a combination of a TLR7/8 agonist and a TLR9 agonist.

Preferably, the TLR agonist is administered at a dose of 1 mg to 8 mg per injection.

In a preferred embodiment, the TLR agonist is administered two times, 7 days and 2 days before sentinel lymph node biopsy (SNB). In another preferred embodiment, the TLR agonist is administered two times, 2 weeks and one week before sentinel lymph node biopsy (SNB).

Preferably, the treatment is combined with an adjuvant therapy selected from a checkpoint inhibitor or an immune-modulatory cytokine, preferably in case of a recurrence.

In a preferred embodiment, the selection of the TLR agonist is based on the patient's immune response profile, determined by measuring dendritic cell activation levels.

Preferably, the selection of the TLR agonist is based on the patient's cytokine expression profile, wherein levels of IL-6, IL-12, TNF, IFNγ, and/or CXCL10 are assessed.

In a preferred embodiment, the selection of the TLR agonist is based on the patient's Toll-like receptor (TLR) expression levels, wherein higherTLR9 expression indicates administration of a TLR9 agonist and higher TLR7/8 expression indicates administration of a TLR7/8 agonist or a combination thereof.

In an embodiment, the intradermal administration is performed in multiple injections surrounding the primary tumor excision site to maximize local immune activation. In another embodiment, the intradermal administration is performed in a single injection immediately adjacent to the tumor excision scar.

In an embodiment, the administration frequency and dose are adjusted based on the patient's baseline immune biomarker levels.

Preferably, the patient is stratified into a high or low responder group based on baseline immune biomarkers, and the selection of the TLR agonist is adjusted accordingly.

In another embodiment, the immune response to the first administration is assessed prior to the second administration, and the selection of the second dose is modified accordingly.

### FIGURES

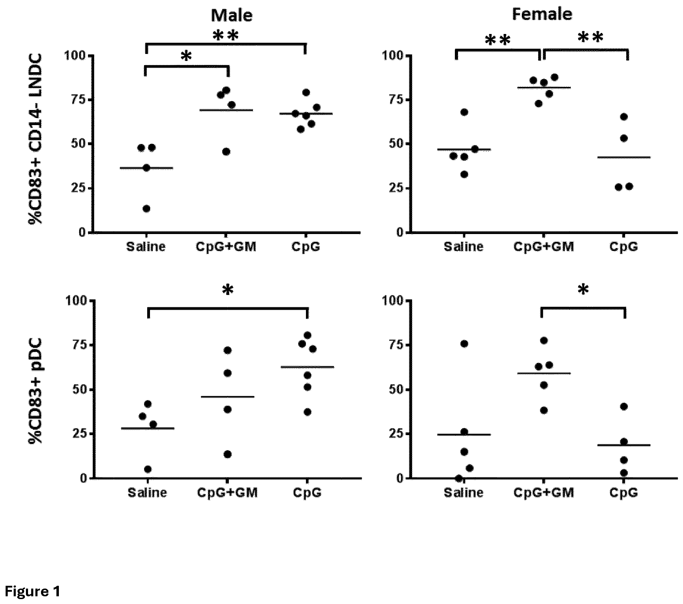

Figure 1. Added local and systemic effects of GM-CSF, co-delivered locally with CpG-B, in early-stage melanoma. Results shown are from patients receiving either a saline placebo (saline), or two administrations of CpG-B (CpG, 1 mg) or CpG combined with GM-CSF (CpG+GM, 1 mg + 100 µg) at day -7 and day -2 before sentinel lymph node biopsy (SNB), or GM-CSF alone (GM-CSF), 4 doses of 3µg/kg divided over the four days leading up to SNB. All were administered at the excision site of the primary tumor. Shown are CD14- lymph node resident conventional DC (cDC) and plasmacytoid (pDC) dendritic cell activation (by CD83) in men and women after local treatment with CpG or CpG+GM.

### DETAILED DESCRIPTION OF THE INVENTION

### Definitions

As used herein, the term "sentinel lymph node biopsy" (SLNB) refers to a surgical procedure for identifying, excising, and analyzing the sentinel lymph node (SLN), which is the first lymph node or group of nodes that receives lymphatic drainage from the primary tumor site. SLNB is performed to assess the presence or absence of metastatic tumor cells within the sentinel lymph node(s), providing diagnostic and prognostic information for cancer staging and treatment decisions.

The procedure comprises:
- Lymphatic Mapping: The injection of a radioactive tracer, a vital dye (such as a blue dye), or both near the tumor site to facilitate identification of the SLN(s).
- Sentinel Lymph Node Identification: The localization of the SLN(s) using a gamma probe, visual inspection, or both to detect the tracer or dye.
- Surgical Excision: The removal of the identified sentinel lymph node(s) for pathological examination.
- Histopathological Analysis: The assessment of the excised node(s) for the presence of tumor cells using standard techniques, including but not limited to hematoxylin and eosin (H&E) staining, immunohistochemistry (IHC), and molecular analysis.

SLNB is typically performed in patients with early-stage melanoma, where it serves as a staging tool to determine whether melanoma has metastasized beyond the primary tumor site. A positive SLNB result (presence of metastatic cancer) indicates a higher risk of systemic disease and may guide further therapeutic intervention, whereas a negative SLNB result (absence of metastatic cancer) suggests a lower risk of disease progression.

For the purposes of this invention, SLNB refers to the standard-of-care procedure as recognized in oncology and surgical practice and includes any clinically accepted modifications or improvements that facilitate the identification, removal, and analysis of the sentinel lymph node(s).

As used herein, the term "Toll-like receptor (TLR) agonist" refers to a compound, molecule, or biological agent that selectively binds to and activates one or more Toll-like receptors (TLRs), leading to the stimulation of innate immune signaling pathways. TLRs are a class of pattern recognition receptors (PRRs) primarily expressed on dendritic cells, macrophages, and other immune cells, where they recognize pathogen-associated molecular patterns (PAMPs) or synthetic analogs thereof.

Upon activation, TLRs initiate intracellular signaling cascades that result in the production of pro-inflammatory cytokines, type-I interferons, and co-stimulatory molecules, thereby enhancing antigen presentation and adaptive immune responses. TLR agonists may include, but are not limited to, nucleic acid-based ligands (e.g., CpG oligodeoxynucleotides), imidazoquinolines, lipopolysaccharide derivatives, and synthetic peptides.

For the purposes of this invention, a TLR agonist refers to any clinically or experimentally validated agent capable of stimulatingTLR-mediated immune activation, including those targeting TLR2, TLR3, TLR4, TLR7, TLR8, TLR9, or combinations thereof.

As used herein, the term "TLR9 agonist" refers to a specific subclass of TLR agonists that selectively binds to and activates Toll-like receptor 9 (TLR9), an intracellular pattern recognition receptor primarily expressed in plasmacytoid dendritic cells (pDCs), B cells, and certain subsets of myeloid dendritic cells.

TLR9 is activated by unmethylated CpG motifs found in bacterial and viral DNA, as well as synthetic CpG oligodeoxynucleotides (CpG-ODNs) designed to mimic these natural ligands. Activation of TLR9 results in a MyD88-dependent signaling cascade, leading to the production of type-I interferons (IFN-α/β), interleukin-12 (IL-12), tumor necrosis factor-alpha (TNF-α), and other pro-inflammatory mediators, thereby enhancing immune surveillance and anti-tumor responses.

For the purposes of this invention, a TLR9 agonist includes, but is not limited to, agatolimod (CPG7909), tilsotolimod (IMO-2125), MGN1703, and other CpG-based immunostimulatory oligonucleotides that function as immune adjuvants or direct immunotherapeutic agents. In a preferred embodiment, the TLR9 agonist for use according to the invention is agatolimod (CPG7909) or tilsotolimod (IMO-2125), both of which have been tested and shown to enhance antigen-presenting cell activation and cytokine release.

TLR9 agonists are known to activate innate immune cells, particularly dendritic cells (DCs), leading to the production of pro-inflammatory cytokines and the initiation of adaptive immune responses. These agonists mimic unmethylated CpG motifs found in bacterial and viral DNA, which are recognized by TLR9 expressed predominantly on plasmacytoid dendritic cells (pDCs) and certain subsets of myeloid dendritic cells. Activation of these cells results in the upregulation of co-stimulatory molecules, secretion of interferon-alpha (IFN-α), and enhancement of antigen presentation to T cells, thereby promoting anti-tumor immunity.

As used herein, the term "TLR7/8 agonist" refers to a specific subclass of Toll-like receptor (TLR) agonists that selectively binds to and activates Toll-like receptor 7 (TLR7) and/or Toll-like receptor 8 (TLR8), which are intracellular pattern recognition receptors primarily expressed in plasmacytoid dendritic cells (pDCs), monocytes, macrophages, and other antigen-presenting cells (APCs).

TLR7 and TLR8 recognize single-stranded RNA (ssRNA) motifs from viral pathogens and synthetic small-molecule ligands. Upon activation, these receptors initiate a MyD88-dependent signaling cascade, leading to the production of type-I interferons (IFN-α/β), pro-inflammatory cytokines (IL-6, IL-12, TNF-α, IFNγ), and chemokines (CXCL10, CCL5), thereby promoting innate immune activation and T cell-mediated immunity.

For the purposes of this invention, a TLR7/8 agonist includes, but is not limited to, imidazoquinolines such as resiquimod (R848), imiquimod, gardiquimod, CL097, and other synthetic or naturally derived small-molecule immune stimulants that activate TLR7 and/or TLR8 pathways. In a preferred embodiment, the TLR7/8 agonist for use according to the invention is resiquimod (R848), which has been demonstrated to activate dendritic cells (DCs) and induce a broad pro-inflammatory cytokine response.

As used herein, the term "primary tumor excision site" refers to the anatomical location from which a primary tumor has been surgically removed, including the immediate surrounding tissue and residual surgical bed. This site represents the area where post-surgical immune modulation, wound healing, and potential residual microscopic tumor presence may influence disease progression and recurrence.

For the purposes of this invention, the primary tumor excision site serves as the target location for administration of a Toll-like receptor (TLR) agonist, where local immune activation is induced prior to sentinel lymph node biopsy (SNB). The term encompasses the surgical wound, peritumoral lymphatic structures, and surrounding stromal tissue, which collectively contribute to antigen uptake, dendritic cell migration, and local cytokine signaling.

The primary tumor excision site may include, but is not limited to:
- Cutaneous or subcutaneous tissue following the removal of a primary melanoma lesion.
- Surgical margins and scar tissue resulting from tumor excision.
- Residual tumor-associated lymphatic vessels that drain into the sentinel lymph node.
- Any surgically exposed tissue at risk of local recurrence or residual tumor cell presence.

For the administration of TLR agonists, the primary tumor excision site may be defined by the original tumor coordinates and perioperative markings or the surgical scar, ensuring precise delivery of immunotherapeutic agents to optimize immune activation within the tumor-draining lymphatic network.

As used herein, the term "checkpoint inhibitor" refers to a therapeutic agent that blocks inhibitory immune checkpoint pathways, thereby enhancing T cell-mediated anti-tumor immune responses. Immune checkpoints are regulatory pathways that modulate immune activation, primarily through co-inhibitory receptors expressed on T cells, which function to prevent excessive immune responses and maintain self-tolerance. However, tumors exploit these pathways to evade immune detection and suppress anti-tumor immunity.

Checkpoint inhibitors function by disrupting inhibitory signaling between immune cells and tumor cells, restoringT cell activation and cytotoxic function. For the purposes of this invention, a checkpoint inhibitor includes, but is not limited to:
- Anti-PD-1 (Programmed Death-1) antibodies, such as pembrolizumab, nivolumab, and cemiplimab.
- Anti-PD-L1 (Programmed Death-Ligand 1) antibodies, such as atezolizumab, durvalumab, and avelumab.
- Anti-CTLA-4 (Cytotoxic T-Lymphocyte-Associated Protein 4) antibodies, such as ipilimumab and tremelimumab.
- Small-molecule inhibitors or engineered proteins targeting immune checkpoint pathways that regulate T cell activation.

For the purposes of this invention, checkpoint inhibitors, immune-modulatory cytokines, and BRAF inhibitors may be administered alone or in combination with TLR agonists, with the objective of enhancing immune activation within the sentinel lymph node and tumor microenvironment.

BRAF inhibitors (e.g., vemurafenib, dabrafenib) target the MAPK pathway, reducing tumor proliferation and, in some cases, enhancing tumor antigen presentation, thereby synergizing with immunotherapies like TLR agonists.

By combining TLR agonists with one or more of these adjuvant therapies, the invention provides a multi-faceted approach to improving anti-tumor immunity and treatment efficacy in early-stage melanoma.As used herein, the term "immune-modulatory cytokine" refers to a biologically active protein or peptide that regulates immune cell function, including the activation, proliferation, differentiation, or suppression of immune responses. Cytokines play a critical role in coordinating innate and adaptive immunity, influencing the strength and duration of anti-tumor immune responses.

For the purposes of this invention, an immune-modulatory cytokine includes, but is not limited to:
- Pro-inflammatory cytokines that enhance T cell activation and antigen presentation, such as interleukin-2 (IL-2), interleukin-12 (IL-12), tumor necrosis factor-alpha (TNF-α), and interferon-gamma (IFNy).
- Type-I interferons, including interferon-alpha (IFN-α) and interferon-beta (IFN-β), which promote dendritic cell maturation and anti-viral immune responses.
- Granulocyte-macrophage colony-stimulating factor (GM-CSF), which enhances antigen presentation by dendritic cells and macrophages.
- Chemokines that regulate immune cell trafficking, such as C-X-C motif chemokine ligand 10 (CXCL10) and C-C motif chemokine ligand 5 (CCL5).
- T cell-stimulating cytokines, including interleukin-15 (IL-15) and interleukin-21 (IL-21), which enhance memory T cell persistence and effector function.

For the purposes of this invention, immune-modulatory cytokines may be administered in combination with TLR agonists to enhance the activation of antigen-presenting cells and T cell priming within the sentinel lymph node microenvironment.

As used herein, the term "Toll-like receptor (TLR) expression level" refers to the amount, density, or activity of Toll-like receptor (TLR) proteins expressed on the surface or within the endosomal compartments of immune cells, including but not limited to dendritic cells, macrophages, monocytes, B cells, and other antigen-presenting cells (APCs). TLR expression levels can be measured at the mRNA level (gene expression) or protein level (surface or intracellular receptor density) and serve as a biomarker for immune responsiveness to TLR agonist-based therapies.

For the purposes of this invention, TLR expression level may refer to:
TLR7, TLR8, or TLR9 expression levels on plasmacytoid dendritic cells (pDCs), myeloid dendritic cells (mDCs), monocytes, or B cells.

Baseline TLR expression prior to treatment, used to assess a patient's predicted responsiveness to a TLR agonist.

Changes in TLR expression following TLR agonist administration, indicating immune activation or modulation.

Quantification methods, including flow cytometry, quantitative PCR (qPCR), RNA sequencing (RNA-seq), immunohistochemistry (IHC), or enzyme-linked immunosorbent assays (ELISA).

For the purposes of this invention, TLR expression level may be used as a biomarker for:
Selecting a TLR9 agonist in patients with high TLR9 expression on dendritic cells.

Selecting a TLR7/8 agonist in patients with elevated TLR7 or TLR8 expression to enhance pDC and monocyte activation.

Determining whether a combination of TLR7/8 and TLR9 agonists is required for optimal immune stimulation.

TLR expression level may be evaluated in whole blood, serum, lymph node biopsies, tumor tissue, or isolated immune cell populations, providing a patient-specific immune profile to guide treatment selection.

### Embodiments

The present invention relates to the use of Toll-like receptor (TLR) agonists in the treatment of early-stage melanoma in male patients. More specifically, the invention provides for the administration of a TLR9 agonist at the primary tumor excision site prior to sentinel lymph node biopsy (SNB) to enhance immune activation, thereby improving the detection and clearance of residual tumor cells within the sentinel lymph node and systemicallyand reducing the risk of recurrence.

The invention is based on the intradermal administration of a TLR9 agonist at the site of primary tumor excision. This localized administration ensures direct immune activation within the tumor-draining lymphatic network, leading to increased antigen uptake and processing by resident dendritic cells in the sentinel lymph node. The treatment is administered in advance of the SNB procedure, allowing sufficient time for immune activation to occur and for antigen-presenting cells to migrate to the sentinel lymph node, where they can effectively prime tumor-specific T cells and possibly NK cells.

In accordance with the invention, the TLR9 agonist may be selected from known CpG oligodeoxynucleotides, including but not limited to agatolimod (CPG7909) or tilsotolimod (IMO-2125). These agonists have been demonstrated to induce potent immune activation in human clinical studies and are suitable for use in melanoma immunotherapy. The selected TLR9 agonist may be formulated as an injectable composition suitable for intradermal administration, comprising the active agent in a pharmaceutically acceptable carrier. The formulation may include additional excipients to stabilize the active ingredient and to optimize its bioavailability at the administration site. Intradermal delivery devices, such as microneedle patches or slow-release systems, may be considered as alternative administration methods to enhance local immune activation and improve patient compliance. These approaches could optimize the controlled release of TLR agonists, ensuring prolonged immune stimulation at the tumor excision site.

The method of administration involves injecting the TLR9 agonist intradermally at the surgical excision site. The dosage may range from 1 mg to 8 mg per injection, depending on the compound or possible therapeutic combinations used.

The administration may be performed as a single injection or as multiple doses administered over a defined period prior to SNB. In a preferred embodiment, the TLR9 agonist is administered at two time points, specifically 7 days and 2 days prior to SNB, to ensure optimal immune activation within the sentinel lymph node. In another preferred embodiment, the TLR9 agonist is administered 7-10 days prior to SNB.

The invention specifically applies to male patients diagnosed with early-stage melanoma. It has been observed that immune responses to TLR9 stimulation differ between male and female patients, with males exhibiting a stronger activation of lymph node-resident dendritic cells (LNR-cDCs) following TLR9 agonist administration. This enhanced response is associated with increased expression of co-stimulatory molecules, higher levels of pro-inflammatory cytokines, and improved antigen presentation, which collectively contribute to a more robust anti-tumor immune response. By selectively applying TLR9 agonist therapy to male patients, the invention optimizes treatment efficacy by leveraging the natural immune response characteristics of this patient group.

The invention may be further combined with other adjuvant immunotherapies to enhance overall treatment efficacy. In one embodiment, the TLR9 agonist is co-administered with an immune checkpoint inhibitor, such as an anti-PD-1 or anti-CTLA-4 antibody, to further potentiate T cell-mediated anti-tumor responses. Additionally, the treatment may be used in combination with standard melanoma therapies, including surgery, targeted therapy, and radiation, as part of an integrated treatment regimen. In a preferred embodiment, the TLR agonist treatment is combined with a checkpoint inhibitor, such as an anti-PD-1, anti-PD-L1, or anti-CTLA-4 antibody, or a conventional DC-activating cytokine like GM-CSF, to enhance T cell priming and anti-tumor immunity.

Overall, the invention provides a novel approach to immunotherapy for early-stage melanoma, utilizing the targeted administration of a TLR9 agonist in male patients to maximize immune activation prior to SNB. By leveraging the natural immune response differences between male and female patients, this method enhances dendritic cell activation, improves antigen presentation, and reduces the likelihood of disease recurrence.

In another aspect, the invention provides aToll-like receptor (TLR) agonists for use in the treatment of early-stage melanoma in female patients, specifically through the administration of a TLR7/8 agonist, alone or in combination with a TLR9 agonist, at the primary tumor excision site prior to sentinel lymph node biopsy (SNB). This approach is designed to enhance immune activation within the sentinel lymph node (SLN) microenvironment, leading to improved antigen presentation, dendritic cell activation, and subsequent priming of anti-tumor T cells.

It has been observed that female patients exhibit differential immune responses to TLR agonists compared to male patients. Specifically, prior studies have shown that TLR9 stimulation alone does not elicit the same level of immune activation in females as it does in males. However, TLR7 and TLR8 agonists induce a more potent immune response in females, particularly by stimulating plasmacytoid dendritic cells (pDCs) and conventional dendritic cell subsets (cDC), leading to a robust secretion of type-I interferons (IFN-α, IFN-β) and pro-inflammatory cytokines (IL-6, IL-12, TNF-α, and IFNγ). These cytokines promote T cell priming and cytotoxic responses that are critical for melanoma control.

In accordance with the invention, the TLR7/8 agonist may be administered alone or in combination with a TLR9 agonist to achieve optimal immune activation. Suitable TLR7/8 agonists include, but are not limited to, resiquimod (R848), imiquimod, gardiquimod, and other imidazoquinoline-based molecules. These agonists function by binding to intracellular TLR7 and TLR8, initiating a MyD88-dependent signaling cascade, which results in the activation of nuclear factor-kappa B (NF-κB) and the production of inflammatory mediators that enhance tumor antigen presentation.

For female patients, the combination of TLR7/8 and TLR9 agonists may be particularly advantageous, as the synergistic activation of both pathways enhances cross-presentation of tumor antigens by dendritic cells and promotes the recruitment of CD8+ cytotoxic T lymphocytes (CTLs) to the sentinel lymph node. The TLR9 agonist, if used in combination, may be selected from agatolimod (CPG7909), tilsotolimod (IMO-2125), or other CpG-based oligonucleotides that specifically stimulate TLR9-expressing immune cells.

The administration of the TLR7/8 agonist, alone or in combination with a TLR9 agonist, is performed intradermally at the primary tumor excision site. This approach leverages the natural drainage of immune cells from the injection site to the sentinel lymph node, ensuring that antigen-presenting cells are activated in the tumor-draining lymphatic system, where they can prime tumor-specific T cells. The TLR7/8 agonist may be formulated as a sterile injectable composition comprising the active agent in a pharmaceutically acceptable carrier, with optional stabilizers or adjuvants to enhance immune stimulation. Intradermal delivery devices, such as microneedle patches or slow-release systems, may be considered as alternative administration methods to enhance local immune activation and improve patient compliance. These approaches could optimize the controlled release of TLR agonists, ensuring prolonged immune stimulation at the tumor excision site.

The recommended dosage for intradermal administration may range from 1 mg to 8 mg per injection, depending on patient-specific factors such as body weight, immune status, and baseline TLR7/8 expression levels, preferably one injection 7-10 days prior to SNB. The administration may follow a two-dose regimen, wherein the first dose is given 7 days prior to SNB and the second dose is administered 2 days prior to SNB, ensuring optimal immune activation at the time of lymph node resection.

The efficacy of the treatment can be evaluated through immunological assays, including flow cytometry analysis of dendritic cell activation markers (CD80, CD86, CD83), cytokine profiling (IL-12, IFNγ, TNF-α, CXCL10), and T cell receptor sequencing to assess antigen-specific T cell expansion. Additionally, histopathological examination of the sentinel lymph node can confirm e.g. increased DC activation and increased content of activated CD8+ T cells and reduced presence of immunosuppressive regulatory T cells (Tregs) following treatment.

By selectively applying TLR7/8 agonist therapy to female patients, the invention provides a targeted immunotherapeutic approach that optimizes immune activation based on sex-specific immune response differences. The use of a TLR7/8 agonist alone or in combination with a TLR9 agonist ensures that the immune system is effectively stimulated in patients who may not respond optimally to TLR9 agonist monotherapy.

The invention may also be combined with immune checkpoint inhibitors (such as anti-PD-1, anti-PD-L1, or anti-CTLA-4 antibodies) to further enhance anti-tumor immunity and improve clinical outcomes. Additionally, the approach may be integrated into standard melanoma treatment regimens, including surgical excision, targeted therapy, and radiation therapy, providing a comprehensive strategy for preventing disease recurrence and improving long-term survival in female melanoma patients.

In a preferred embodiment, the TLR9 agonist for use according to the invention is selected from agatolimod (CPG7909) or tilsotolimod (IMO-2125). These CpG oligodeoxynucleotides have been demonstrated to enhance dendritic cell activation, upregulate co-stimulatory molecules (CD80, CD86), and induce pro-inflammatory cytokine production (IL-12, TNF-α, and IFNγ). In melanoma patients, TLR9 stimulation has been shown to prime an adaptive immune response by increasing the number of activated lymph node-resident dendritic cells (LNR-cDCs) and enhancing cross-presentation of tumor antigens. The selection of a specific TLR9 agonist is preferably based on stability, immunogenic potency, and suitability for intradermal administration to ensure optimal immune activation.

In a preferred embodiment, the TLR7/8 agonist for use according to the invention is selected from resiquimod (R848), imiquimod, gardiquimod, or other imidazoquinoline-based compounds. These agonists preferably activate plasmacytoid dendritic cells (pDCs) and conventional dendritic cells (cDCs), leading to a robust secretion of type-I interferons (IFN-α, IFN-β) and pro-inflammatory cytokines (IL-6, TNF-α, IFNγ). Studies have shown that TLR7/8 agonists preferably induce pDC maturation and IFNα production, which are critical for promoting antigen presentation and enhancing CD8+ T cell priming against tumor-associated antigens.

In a particularly preferred embodiment, the combination of a TLR7/8 agonist and a TLR9 agonist for use according to the invention is used, as it results in synergistic activation of both LNR-cDCs and pDCs, leading to enhanced antigen uptake, cross-presentation, and recruitment of tumor-specific cytotoxic T cells within the sentinel lymph node. The combination approach has been shown to increase dendritic cell activation markers (CD83, CCR7), promote a pro-inflammatory cytokine environment (CXCL10, IL-12), and enhance IFNγ responses, leading to greater expansion of tumor-reactive T cells in the sentinel lymph node.

The dosage and administration schedule is preferably adapted based on patient-specific factors. In a preferred embodiment, the TLR9 agonist, TLR7/8 agonist, or combination for use according to the invention is administered at a dose of 1 mg to 8 mg per injection, depending on patient body weight, baseline immune status, and tumor characteristics. Preferably one injection is given 7-10 days prior to SNB. In a particularly preferred embodiment, the agonist is administered according to a two-dose schedule, wherein the first dose is administered 7 days before sentinel lymph node biopsy (SNB) and the second dose 2 days before SNB, allowing for optimal immune activation at the time of surgery.

In a preferred embodiment, the TLR9 agonist, TLR7/8 agonist, or a combination thereof for use according to the invention is administered at a dose of 1 mg to 8 mg per injection, depending on patient body weight, immune response profile, and treatment objectives.

Preferably, for male patients, the TLR9 agonist is administered as a single injection of 8 mg one week prior to sentinel lymph node biopsy (SNB) to enhance dendritic cell activation and antigen presentation. Alternatively, in a particularly preferred embodiment, the TLR9 agonist is administered at a dose of 1 mg in a two-dose regimen, wherein the first dose is given 7 days before SNB and the second dose 2 days before SNB, allowing for sustained immune activation at the time of surgery.

In a further preferred embodiment, for female patients, the TLR7/8 agonist, alone or in combination with a TLR9 agonist, is administered intradermally at the tumor excision site. Preferably, the TLR7/8 agonist is administered at a dose of 1 mg to 8 mg, either as a single injection 7-10 days before SNB or according to a two-dose schedule at 7 and 2 days before SNB. In a particularly preferred embodiment, the combination of a TLR7/8 agonist and a TLR9 agonist is co-administered with an immune-modulatory cytokine, such as GM-CSF at a dose of 100 µg, to enhance dendritic cell activation in female patients.

In an alternative embodiment, the TLR agonist treatment is combined with GM-CSF alone, administered at 3 µg/kg per day for four consecutive days before SNB, to induce migration and activation of skin-derived dendritic cells.

In a further preferred embodiment, intradermal administration is performed using microneedle patches or slow-release systems to optimize local immune activation and improve patient compliance.

In an additional preferred embodiment, the selection of the dosage regimen is based on patient-specific immune biomarkers, wherein patients are stratified into high and low responder groups, and the frequency and combination of TLR agonists are adjusted accordingly to maximize therapeutic efficacy.

In a preferred embodiment, the TLR9 agonist, TLR7/8 agonist, or combination for use according to the invention is administered in combination with an immune checkpoint inhibitor, selected from anti-PD-1, anti-PD-L1, or anti-CTLA-4 antibodies. The combination of TLR agonists and checkpoint inhibitors has been demonstrated to preferably enhance T cell activation, reduce immunosuppressive regulatory T cells (Tregs), and increase the infiltration of effector CD8+ T cells in the sentinel lymph node microenvironment, improving the likelihood of sustained anti-tumor immunity.

In a preferred embodiment, the TLR9 agonist, TLR7/8 agonist, or combination for use according to the invention is administered together with an immune-modulatory cytokine, such as interleukin-12 (IL-12), tumor necrosis factor-alpha (TNF-α), interferon-gamma (IFNγ), granulocyte-macrophage colony-stimulating factor (GM-CSF), or other cytokines that enhance antigen presentation and T cell priming. The inclusion of such cytokines preferably further potentiates dendritic cell activation, enhances cross-presentation of tumor antigens, and improves CD8+ T cell proliferation in the sentinel lymph node.

In a preferred embodiment, the TLR9 agonist, TLR7/8 agonist, or combination for use according to the invention is administered intradermally at the primary tumor excision site to preferably target the tumor-draining lymphatic system, where antigen-presenting cells are naturally recruited. In a particularly preferred embodiment, the administration is performed in multiple injections surrounding the excision site to ensure optimal immune cell uptake and activation.

In a further preferred embodiment, the TLR9 agonist, TLR7/8 agonist, or combination for use according to the invention is administered peritumorally instead of intradermally, particularly in cases where the primary tumor site remains intact, or when residual tumor cells are suspected to be present within the tumor bed. This route of administration has been demonstrated to preferably increase local immune cell infiltration, promote tumor antigen release, and enhance dendritic cell-mediated cross-presentation.

In a preferred embodiment, the selection of the TLR9 agonist, TLR7/8 agonist, or combination for use according to the invention is based on patient-specific immune biomarkers, including dendritic cell activation levels, cytokine expression profiles, and TLR7, TLR8, or TLR9 expression levels. The use of immune profiling to guide treatment selection ensures that patients preferably receive the most effective immunotherapy strategy tailored to their baseline immune status.

In a further preferred embodiment, the selection of the TLR9 agonist, TLR7/8 agonist, or combination for use according to the invention is determined based on immune response to an initial administration, wherein the first dose is used to evaluate cytokine induction, dendritic cell maturation, and T cell priming, and subsequent doses are preferably adjusted accordingly.

In a particularly preferred embodiment, patients are stratified into high and low responder groups based on baseline immune biomarker levels, wherein high responders preferably receive a single-agent TLR agonist, while low responders preferably receive combination therapy with a TLR7/8 and TLR9 agonist or additional immune-stimulatory agents.

These embodiments collectively ensure that the TLR9 agonist, TLR7/8 agonist, or combination for use according to the invention is optimized for patient-specific immune responses, administered in a manner that enhances antigen presentation within the sentinel lymph node, and effectively stimulates an anti-tumor immune response to improve clinical outcomes in early-stage melanoma patients.

### EXAMPLES

### Example 1

In localized melanoma, disease recurrence after resection of the primary tumor is dependent on disease stage, which includes risk factors such as Breslow thickness, tumor ulceration and whether or not the tumor has metastasized to the regional lymphatics. As a prognostic measure, a sentinel node biopsy (SNB) is performed to assess lymphogenic metastasis, although so far the treatment implications are very limited after identifying a tumor positive (sentinel) lymph node (stage III disease). A complete lymph node dissection is no longer indicated as this does not improve overall survival.

In three randomized controlled phase II trials, we intradermally injected GM-CSF alone, the TLR9 agonist CpG-B/CPG7909 alone,4,5 or CpG-B/CPG7909 and GM-CSF combined directly adjacent to the excision scar of the primary tumor in early-stage (i.e. localized) melanoma in the week leading up to the SNB. In the first trial, patients received four consecutive daily doses of 3 µg/kg body weight GM-CSF (n = 6) or placebo (n = 6), immediately preceding SNB. In the second trial patients received one dose of 8 mg CpG-B (n = 11) or placebo (n = 13) one week prior to SNB, and in the third trial, patients received 1 mg CpG-B (n = 10), 1 mg CPG-B with 100 µg GM-CSF (n = 9) or placebo (n = 9), 7 and 2 days prior to SNB.

### Myeloid subset modulation and sex disparities

We have identified two skin-derived migratory conventional DC subsets (cDC), i.e. Langerhans cells (LC) and dermal dendritic cells (DDC), and two lymph node resident cDC subsets (LNR-cDC) in melanoma SLN. The LNR-cDC subsets differ in their expression of CD14, with a subpopulation of the CD14- LNR-cDC phenotypically resembling CD141 + cDC1 with particular cross-presenting abilities. We found locally administered GM-CSF to activate and induce the migration of skin-derived cDC subsets to the SLN. The combination of GM-CSF with CpG-B also resulted in superior activation of skin derived cDC. These findings are in keeping with increased activation of skin explant-emigrated cDC subsequent to intradermal GM-CSF injection . In contrast, no such effect was observed for CpG-B, but rather cDC1 and pDC were mobilized to the deep dermis upon intradermal delivery of CpG-B. We have also reported that the addition of GM-CSF to locally administered CpG-B prior to SNB, resulted in more profound activation of the LNR-cDC subsets, and indeed increased the cross-presentation capacity of SLN-derived leukocyte suspensions.

Remarkably, when we subdivided patients by sex, disparities in terms of DC activation became apparent (see Figure 1). Whereas CpG-B monotherapy in men induced significant activation of both CD14- LNR-cDC and pDC in the SLN (by CD83 expression), and in equal measure as when combined with GM-CSF, in women an equivalent activation induction in these subsets required combined CpG-B and GM-CSF activation. Of note, similar observations were made for the CD14+ LNR-cDC subset. The role of sex specific hormones and genetics on the immune system and cancer progression has been well documented, but this has not yet led to cancer treatment implications based on the patient's sex. Our data suggest that optimal activation of DC and, as a consequence, subsequent priming of antitumor effector T cells in women with early-stage melanoma may require combined administration of GM-CSF and CpG-B. This may be related to sex-dependent differences in TLR9 expression levels as previously observed in a mouse study, wherein increased TLR9 expression levels in males led to improved clearance of a viral infection. Moreover, a sex-dependent inflammatory cytokine pattern during melanoma development was also described before in an experimental mouse model. Improved pDC activation and IFNα release by GM-CSF has also been reported, but, as far as we know, has never previously been linked to sex-dependent mechanisms. It is important to note that despite these remarkable differences in DC subset activation, we did not find any differences in recurrence-free survival (RFS) between women receiving CpG-B only or CpG-B combined with GM-CSF.

### Example 2

Here, we report on sex-based differences in the immune response after the local administration of the TLR9 agonist agatolimod/CPG7909. In an effort to devise a more effective LNR-DC activating therapy for women, we compared the effects of in vitro stimulation of SLN-derived cells from female donors with CPG7909 and the TLR7/8 agonist resiquimod/R848 .

### Methods

In a randomized Phase-2 trials comparing CPG7909 to a saline placebo (ISRCTN63321797), 43 patients with early-stage melanoma were assessed for post-treatment sex-based immunological differences. Next, activation of DCs by flow cytometry and changes in cytokine- and chemokine expression by CBA were evaluated after a 2-day in-vitro stimulation with CPG7909 and R848, using single-cell suspensions of healthy donor lymph nodes (obtained from prophylactic mastectomies n=5) and melanoma SLN (n=5), from females.

### Results

Upon i.d. CPG7909 delivery at the primary tumor excision site, both migratory and LNR-cDC subsets in SLN displayed a higher activation state in men as compared to women based on expression levels of CD80 and CD83. Whereas in-vivo systemic type-1 IFN response signatures and TNF levels upon in-vitro CPG7909 re-stimulation of SLN single-cell suspensions were similar in men and women, in-vitro release levels of IL-1β, TNF, and IL-6, all known as contributors to cDC activation, were more robust in men, as were Th1/Th2 cytokine levels upon in-vitro CD3/CD28-mediated polyclonal activation. Comparative testing of CPG7909 and the TLR7/8 agonist R848 in 2-day cultures of both healthy and sentinel lymph nodes from women showed R848 to induce superior activation of cDC subsets with significantly higher levels of CD80, PD-L1 and CD40. Whereas CPG7909 induced higher levels of IFNα release, R848 induced higher levels of TNF, IL-6, IL-10, IL-12, IFNγ, and CXCL10. Correlation matrix analyses indicated a role for IFNα, TNF and IL-6 in activating LNR-cDC upon CPG7909 exposure, whereas R848-induced LNR-cDC activation appeared to be more cytokine-independent. While CPG7909-induced IL-10 seemed to counter LNR-cDC activation, R848-induced IL-10 seemed to be less effective in this regard.

By combining a TLR9 with a TLR7/8 agonist for local immune modulation in early-stage melanoma, full-range DC subset activation in the melanoma SLN is assured with robust pro-inflammatory T-cell activating and recruiting activity, in men and women alike.

### MATERIALS AND METHODS

### Clinical study design and clinical procedures

Viable SLN cells were collected from 43 patients with early-stage melanoma who had previously received treatment with intradermal CpG7909 monotherapy or saline between June 2004 and June 2007 in two single-blinded and placebo-controlled randomized trials. All patients received CpG7909 injections (8 mg in trial 1 and 1 mg in trial 2) at the tumor excision site one week before the SNB, and another one of 1 mg in trial 2 two days before SNB, followed by wide local excision (WLE). In addition treatment naive SLN single-cell suspensions, obtained from 5 female patients with early-stage melanoma who underwent a SNB and WLE at Amsterdam UMC between February 2018 and March 2021 were selected. Additionally, axillary HLN were obtained from 5 BRCA-1 or - 2 positive female patients undergoing a prophylactic mastectomy in the Antoni van Leeuwenhoek Hospital between January 2012 and September 2014. Single-cell suspensions from both HLN and melanoma SLN of all female participants were *in vitro* stimulated with the TLR7/8 agonist R848/resiquimod and the TLR9 agonist CpG7909/agatolimod.

### Lymph node handling and sampling

All SLN were identified and retrieved by the standard triple technique consisting of preoperative lymphoscintigraphy, blue-dye injection, and intraoperative lymphoscintigraphy with a hand-held gamma-detecting probe. Immediately after removal, SLN were collected in a dry sterile container and taken to the pathology department of the VUmc for retrieval of viable cells under sterile conditions. Before routine histological examination and after confirmation by the pathologist that the SLN was suitable for harvesting (i.e. >0·5 cm), viable cells were scraped from the SLN using a previously described method, without interfering with standard diagnostic procedures (Vuylsteke RJ et al. Am J Pathol. 2002;161(1):19-26). In short, after measuring the size of the SLN, it was bisected crosswise with a surgical scalpel and the cutting surface of the SLN was scraped 10 times with a surgical blade (size no. 22, Swann Morton Ltd., Sheffield, England). SLN cells were rinsed from the blade with medium containing 0.1% DNAse, 0.14% collagenase A (Boehringer), 5% fetal calf serum (FSC), and penicillin-streptomycin L l-glutamine (PSG), then transferred to a sterile flask, and subsequently incubated for 30-45 min at 37°C. Finally, collected SLN cells were washed twice in culture medium (CM), comprising Iscove's modified Dulbecco's medium (IMDM; BioWhittaker, Verviers, Belgium), supplemented with 10% heat-inactivated FCS, PSG and gentamycin. The obtained viable SLN single-cell samples were either immediately used for *ex-vivo* immune monitoring or cryostored until use for *in-vitro* culture with TLR agonists. After SLN cell sampling the bisected SLN was further processed by the pathologist according to the SLN protocol of the pathology department of Amsterdam UMC, location VUmc. Axillary HLN were retrieved from prophylactic mastectomy specimen in the Antoni van Leeuwenhoek Hospital. No additional skin incision or radio isotope or patent blue injection was used. The HLN were collected in a sterile test-tube containing complete medium (CM) and transported to Amsterdam UMC, location VUmc. HLNs were cut into 2-mm3 pieces and further processed into single-cell suspensions using the same dissociation method as described above. Harvested cells were cryopreserved until use for *in-vitro* cultures with TLR agonists.

### Lymph node cultures

Collected SLN or HLN cells were plated in a 48-well plate at 5 x 10⁵ cells/well and cultured for two days in CM or CM supplemented with either 5 µg/ml CPG7909 (Coley Pharmaceutical Group, Wellesley, Massachusetts, USA) or 10 µg/ml R848 (InvivoGen, San Diego CA). After two days of culture, 100 µl supernatant from each well was removed and stored at -20°C for cytokine release assessment. SLN cells were harvested from the wells and subsequently washed and resuspended in 100 µl FACS buffer (phosphate-buffered saline [PBS] with 0.1% bovine serum albumin (BSA) and 0.02% sodium azide [NaN3]) for subsequent flowcytometric phenotyping.

### Flowcytometric phenotyping

Four-color flow cytometry of freshly isolated SLN cell suspensions from the clinical trials was performed as previously described (van de Ven R et al. Blood. 2011;118(9):2502-10.). For methods and statistics we refer to previously published work (Molenkamp BG et al. Clin Cancer Res. 2008;14(14):4532-42, Sluijter BJ et al. Cancer Immunol Res. 2015;3(5):495-505).

Available FACS data were (re)analyzed in a separate comprehensive analysis exploring DC maturation and activation state compared to placebo, and subsequently assessed for sex disparities.

For *in-vitro* stimulation of single-cell suspensions derived from HLN and SLN with R848 and CpG, the following monoclonal antibodies (mAbs) were used in 8-color panels: CD1a-FITC, CD14-PerCP-Cy5.5, CD1c-PE-Cy7, CD11c-APC, CD45-AF700, CD19-PE-CF594, CD83-PE, CD80-BV421, PD-L1-BV786, CD40-BV711, CD123-BV65. Frequency and activation state of DC subsets were assessed by membrane staining: LN cell suspensions were stained in FACS buffer and incubated with mAbs for 30 min at 4°C. After incubation, cells were washed in FACS buffer to remove excess antibodies and used for flowcytometric analysis. Fluorescence Minus One (FMO) negative controls were included. Per measurement a minimum of 2 x 10⁵ cells were required. Multicolor flow cytometry was performed using the BD LSR Fortessa. Data were analyzed using FlowJo (v.10.7.1) analysis software.

### Type-1 IFN response transcript analysis

Total RNA was isolated from pre- and post-treatment peripheral blood mononuclear cells (PBMCs) from patients participating in the CPG7909 clinical trials and reverse transcribed as previously described (Koster BD et al. Oncoimmunology. 2020;9(1):1708066.). Transcript levels of IFN response genes (IRGs) in PBMC were analyzed before treatment (pre-treatment; t=0), one week after treatment (t=7) and three weeks after treatment (t=21). Post-treatment IRGs were defined as the maximum IRG expression levels at any of the time points t=7 or t=21 days. For a list of the 33 IRGs included in the analysis we refer to Koster *et al.* (Koster BD et al. Oncoimmunology. 2020;9(1):1708066).

### Cytokine profiling

SLN inflammatory and T cell cytokine detection in SLN single-cell suspension culture supernatants from in-vivo CPG7909- or placebo-treated patients were previously described ((Molenkamp BG et al. Clin Cancer Res. 2008;14(14):4532-42, Sluijter BJ et al. Cancer Immunol Res. 2015;3(5):495-505); van den Hout et al Cancer Immunol Immunother 2016). Released cytokines from SLN and HLN cultures at day 2 of culture with either CM, CPG7909 or R848 were measured by cytometric bead array (CBA) analysis using a human Flex kit (TNF, IL-6, IL-10, IL-12p70, CXCL10, IFNγ). CBA analyses were performed following the manufacturer's instructions and analyzed by flow cytometry (all from BD Biosciences). Concentration in the supernatants (pg/mL) of the respective cytokines and chemokine was calculated using FCAP array software (Soft Flow Hungary Ltd.). A human IFN-α platinum enzyme-linked immunosorbent assay (ELISA) kit (catalog number BMS216CE; ThermoFisher Scientific) was used to determine IFN-α levels after *in vitro* TLR stimulation according to the manufacturer's instructions. The Synergy^{™} HT spectro-photometer was used to read the plate.

### Statistical analysis

Normal distribution was assessed by means of the Shapiro-Wilk normality test. Differences between placebo and CpG7909 treatments, for both female and male patients, were evaluated for statistical significance using an unpaired (two-tailed) t-test when normally distributed or the Mann-Whitney test when non-normally distributed. To asses differences in immune subsets and cytokine/ chemokine levels, between medium condition and treatment (CpG7909 and R848) results were analyzed by the paired t-test (two-tailed) when parameters showed a normal distribution or alternatively analyzed by the non-parametric Wilcoxon test. Similarly, for comparisons between CpG7909 and R848 treatment groups the paired T test (two-tailed) or Wilcoxon test was used. A correlation analysis was performed using R V.4.0.3. The correlation coefficients were calculated using the Spearman rank-order correlation analysis. Differences and correlations were considered statistically significant when P ≤ 0.05. Significance is presented as p<0.05*, <0.01**, <0.001***, 0.0001****. Data were plotted and analyzed using GraphPad Prism software (version 8), and (correlation matrix) heatmaps were created using R (version 4.3.2).

### RESULTS Local CPG7909 delivery in two randomized Phase II studies: patient-related clinical data

In total 43 patients with early-stage melanoma, enrolled in two randomized phase II trials, received intradermal injections around the primary tumor excision site of either CPG7909 (n=21; 9 female and 12 male patients) or a saline placebo (n=22; 9 female and 13 male patients) and were selected to retrospectively assess sex disparities in terms of DC subset activation and pro-inflammatory modulation of the SLN. At baseline, there were no significant differences in clinical tumor characteristics between the treatment and placebo groups for either sex (Table 1).

| **Table 1 Clinical and pathological characteristics of patients treated with TLR9 agonist CpG 7909 vs. Placebo (n=43)** | | | | | | | |
|---|---|---|---|---|---|---|---|
| | | **Female** | | | **Male** | | |
| | | **CpG (n=9)** | **placebo (n=9)** | **p value^{¥}** | **CpG (n=12)** | **placebo (n=13)** | **p value^{¥}** |
| Age (years) | Mean ± SD | 53·3 ± 12·7 | 47·8 ± 10·5 | 0·340 | 55·7 ± 12·7 | 57·4 ± 12·9 | 0·437 |
| Melanoma site | Head and Neck | 1 (11%) | 0 | 0·415 | 1 (8%) | 0 | 0·242 |
| | Trunk | 2 (22%) | 4 (44%) | | 10 (83%) | 0 | |
| | Upper extremities | 0 | 0 | | 1 (8%) | 3 (23%) | |
| | Lower extremities | 6 (67%) | 5 (56%) | | 0 | 2 (15%) | |
| Histological subtype | Superficial spreading melanoma (SSM) | 16 (76%) | 8 (89%) | 0·576 | 10 (83%) | 9 (69%) | 0·645 |
| | Nodular | 0 | 0 | | 2 (17%) | 4 (31%) | |
| | Other | 3 (33%) | 1 (11%) | | 0 | 0 | |
| Breslow (mm) | Median [IQR] | 1-4 [0·9-2·1] | 1·2 [0·5-1·7] | 0·258 | 1·6 [0·9-2·2] | 1-6 [1·0-3-0] | 0·168 |
| Ulceration | Yes | 1 (11%) | 1 (11%) | 1·000 | 3 (25%) | 3 (23%) | 1·000 |
| | No | 8 (89%) | 8(89%) | | 9 (75%) | 10 (77%) | |

| | | | | | | | |
|---|---|---|---|---|---|---|---|
| Data are n (%) of median (IQR]. Percentages may not total 100 because of rounding. T category is according to TNM staging. ^{¥}Mann-Whitney U test was used to compare continuous variables between different patient groups; the X² test or Fisher's exact test was used to assess associations between two categorical variables | | | | | | | |

In terms of clinical efficacy, men benefitted more from i.d. CPG7909 than women, with significantly lower tumor-positive SLN rates and significantly improved recurrence-free survival (RFS) at a median follow-up of 88.8 months -although, of note, women seemingly had lower tumor-positive SLN rates and better RFS even without CPG7909 treatment.

### Sex-based differences in DC subset activation after local treatment with CPG7909 vs. placebo in early-stage melanoma

Maturation and activation state of conventional DC (cDC) subsets in SLN after intradermal delivery of the TLR9 agonist CPG7909 were assessed by means of fluorescence-activated cell sorting (FACS) analysis, as previously described (van den Hout et al Cancer Immunol Res 2017). cDC subsets in the SLN comprise two migratory CD1a+ cDC subsets (i.e., CD1a+CD11cintCD1ahi Langerhans cells [LCs] and CD1 a+CD11 chiCD1 aint dermal-like DCs [dDCs]) and two lymph node-resident (LNR) CD1a- cDC subsets (i.e., CD1a-CD11c+CD14- and CD1a- CD11c+CD14+ LNR-cDC, the latter consisting mostly of cDC2 and for upto 25% of cDC1. Concertedly high expression levels of the maturation and activation markers CD83 and CD80 were consistently observed post-treatment in all four cDC subsets in men. In contrast, consistently lower levels were observed in women. As cDC activation was assumed to be induced by IFNα released by pDC upon their direct activation by CPG7909, we also compared their post-treatment activation between men and women and found significantly higher levels of the CD83 and CD80 activation markers in men. However, comparing pre- and post-treatment type-I IFN response signatures, based on expression levels of 33 IFN response genes in PBMC, IFN responses were found to be induced to a similar extent in men and women, negating differences in IFNα release as a root cause for the observed difference in cDC subset activation. Assessment of CPG7909-induced expression of other pro-inflammatory cytokines in overnight cultures of SLN-derived single-cell suspensions showed a more significant and consistent release of TNF, IL-6, IL-10, and IL-1β in men over women. This suggests the possible involvement of CPG7909-triggered cytokines other than IFNα in the observed superior cDC activation in men.

### Sex-based disparities in T-cell cytokine profiles after i.d. treatment with CPG7909

To determine differences in T-cell differentiation in the SLN between men and women after local delivery of CPG7909, SLN-derived single-cell suspension underwent CD3- and CD28-mediated polyclonal stimulation. After 24h supernatants were harvested and T-cell effector cytokines measured. A superior effector T-cell activation was observed in men, evidenced by significantly higher induction of IFNγ, IL-2, IL-5 and IL-10 release levels.

### Comparative assessment of the in-vitro immune modulatory effects of R848 versus CPG7909 in HLN and SLN of women

As CPG7909 clearly favored SLN cDC and T-cell activation in men, we set out to explore the relative efficacy of the TLR7/8 agonist in this respect as therapeutic alternative for women. TLR7 triggering was previously reported to induce superior type-I IFN responses in women as compared to men through activation of pDC. We confirmed relatively high expression levels of TLR7 and TLR9 transcripts in pDC; in contrast, LNR-cDC expressed relatively high levels of TLR8. We cultured single-cell suspensions derived either from HLN (n=5) or melanoma SLN (n=5) from women in complete medium alone or supplemented with either CPG7909 (5 µg/ml) or R848 (10 µg/ml) over a period of two days, after which supernatants were collected and pDC and cDC activation state was assessed by flow cytometry. Table 2 shows the relevant clinical characteristics of the healthy donors and melanoma patients from whom HLN and SLN single-cell suspensions were derived for the in-vitro TLR stimulation cultures. As similar trends were observed for the HLN and SLN, their data were pooled to increase statistical power. Of note, we omitted the CD14+ LNR-cDC subset from these analyses as its frequency after culture was too low for reliable assessment; the CD14- LNR-cDC subset will hereafter be referred to as LNR-cDC. Whereas CPG7909 and R848 induced similar phenotypic activation levels in pDC, R848 induced superior activation in all cDC subsets, and dDC and SLN-cDC in particular, based on CD80, PD-L1 and CD40 expression levels. Of note, CPG7909 did selectively induce increased expression of the maturation marker CD83 on all DC subsets. Cytokine release profiles showed significantly higher levels of IFNα upon CPG7909 exposure, whereas significantly higher levels of TNF, IL-6, and IFNγ were observed upon R848 conditioning. Also higher levels of CXCL10, IL-10, and IL-12p70 were observed in the R848 cultures, although these did not reach statistical significance in a direct comparison to CPG7909. In a small group of men (n=3) we also performed a head-to-head comparison of CPG7909 to R848 in 2-day melanoma SLN single-cell suspension cultures and found similar effects in terms of pDC/cDC activation and cytokine/chemokine release as those observed in women.

### Correlation matrix analyses

Correlation matrix analyses were performed to compare co-regulation of cytokines, chemokines, and DC markers between CPG7909 and R848, and try and identify possible differential mechanisms underlying cDC subset activation. In CPG7909-conditioned lymph nodes there appeared to be a dichotomy between IFNα on the one hand and IL-6 and TNF on the other, each clustering with different cDC activation markers: whereas the former mostly clustered and was more strongly correlated with the B7 family members CD80 and PD-L1, the latter mostly clustered and showed stronger positive correlations with CD40 and CD83. In the R848-modulated lymph nodes IFNα did co-cluster with the other pro-inflammatory cytokines, but the majority of cDC activation markers, including CXCL10, clustered separately from these cytokines, revealing a more cytokine-independent cDC activation mechanism for R848. Based on relative TLR expression patterns, most likely this would have entailed direct TLR8-mediated activation. Whereas IL-10 expression levels were consistently negatively correlated to expression of the B7 family members in the CPG7908-conditioned lymph nodes, this was far less the case for R848, with IL-10 even positively correlating with PD-L1 levels on dDC and LNR-cDC. This raised the question whether this apparent difference in IL-10 responsiveness could have explained the lower activation levels of cDC observed upon CPG7909 exposure. We previously observed enhanced *in-vitro* CPG7909-mediated cDC and pDC activation in SLN from patients with breast cancer upon STAT3 inhibition by AG490, an upstream JAK2-inhibitor. As JAK2/STAT3 inhibition is known to interfere with IL-10R signaling, we here also tested the effects of this combinatorial treatment on pDC, dDC, and LNR-cDC in melanoma SLN and HLN cultures. Rather than up-regulating activation markers on the cDC subsets, addition of the JAK2 inhibitor AG490 did not significantly alter their expression levels. In pDC there was even a trend for down-regulating CD80 and PD-L1, reaching significance only for CD40. Notably, while IL-6 and IL-10 release levels both went down, IFNα release also dropped significantly, possibly accounting for the failure of AG490 to increase CPG7909-induced cDC activation.

## Claims

1. A Toll-like receptor (TLR) agonist selected from a TLR9 agonist for use in the treatment of early-stage melanoma in a male patient, wherein the treatment comprises intradermal administration of the TLR9 agonist at the primary tumor excision site prior to sentinel lymph node biopsy (SNB).

2. A Toll-like receptor (TLR) agonist selected from a TLR7/8 agonist, alone or in combination with a TLR9 agonist, for use in the treatment of early-stage melanoma in a female patient, wherein the treatment comprises intradermal administration of the agonist(s) at the primary tumor excision site prior to sentinel lymph node biopsy (SNB).

3. The TLR agonist for use according to claim 1 or 2, wherein the TLR9 agonist is selected from agatolimod (CPG7909) or tilsotolimod (IMO-2125).

4. The TLR agonist for use according to claim 2, wherein the TLR7/8 agonist is resiquimod (R848).

5. The TLR agonist for use according to claim 2 or 3, wherein the female patient receives a combination of a TLR7/8 agonist and a TLR9 agonist.

6. The TLR agonist for use according to any one of claims 1 to 5, wherein the agonist is administered at a dose of 1 mg to 8 mg per injection.

7. The TLR agonist for use according to any one of claims 1 to 6, wherein the agonist is administered two times, 7 days and 2 days before sentinel lymph node biopsy (SNB).

8. The TLR agonist for use according to any one of claims 1 to 7, wherein the treatment is combined with an adjuvant therapy selected from a checkpoint inhibitor, an immune-modulatory cytokine or a BRAF inhibitor.

9. The TLR agonist for use according to any one of claims 1 to 8, wherein the selection of the agonist is based on the patient's immune response profile, determined by measuring dendritic cell activation levels.

10. The TLR agonist for use according to any one of claims 1 to 9, wherein the selection of the agonist is based on the patient's cytokine expression profile, wherein levels of IL-6, IL-12, TNF, IFNγ, and/or CXCL10 are assessed.

11. The TLR agonist for use according to any one of claims 1 to 10, wherein the selection of the agonist is based on the patient's Toll-like receptor (TLR) expression levels, wherein higher TLR9 expression indicates administration of a TLR9 agonist and higher TLR7/8 expression indicates administration of a TLR7/8 agonist or a combination thereof.

12. The TLR agonist for use according to any one of claims 1 to 11, wherein the intradermal administration is performed in multiple injections surrounding the primary tumor excision site to maximize local immune activation.

13. The TLR agonist for use according to any one of claims 1 to 12, wherein the administration frequency and dose are adjusted based on the patient's baseline immune biomarker levels.

14. The TLR agonist for use according to any one of claims 1 to 13, wherein the patient is stratified into a high or low responder group based on baseline immune biomarkers, and the selection of the agonist is adjusted accordingly.

15. The TLR agonist for use according to any one of claims 1 to 14, wherein the immune response to the first administration is assessed prior to the second administration, and the selection of the second dose is modified accordingly.
